# EUROPEAN PATENT APPLICATION

(11) **EP 1 454 611 A2**
(43) Date of publication of application: **08.09.2004**
(21) Application number: 04075663.7
(22) Date of filing: 03.03.2004
(51) Int. Cl.: A61K 7/02, A61K 7/035

(54) **Coated cosmetic powder**

(30) Priority: 04.03.2003 IT MI20030383
(71) Applicant: Intercos S.p.A., 20122 Milano (IT)
(72) Inventor: Maio, Giuseppe, 20080 Zelo Surrigone MI (IT); Rando, Pietro, 20090 Opera MI (IT)
(74) Representative: Mittler, Enrico

(57) **Abstract**

The present invention refers to a coated cosmetic powder, preferable for coloured cosmetic products, in particular coated with certain siloxane resin polymers, and also refers to a cosmetic composition which contains said coated cosmetic powder.

The cosmetic powder is constituted by a powder phase coated with silsesquioxane homopolymers and/or copolymers having an average siloxane unit of the general formula R¹ₙSiO_{(4-n)/2}, where each R¹ is independently chosen between a hydrogen atom and a monovalent hydrocarbon group comprising 1 to 10 carbon atoms, wherein more than 80 mole % of R¹ are hydrocarbon groups having from 3 to 10 carbon atoms, n is a value from 1.0 to 1.4, more than 60 mole % of the copolymer comprises R¹SiO_{3/2} units, and having a hydroxyl or alkoxy content from 0.2 to 10% by weight.

## Description

The present invention refers to a coated cosmetic powder, preferably for coloured cosmetic products, in particular coated with certain siloxane resin polymers, and also refers to a cosmetic composition which contains such a coated cosmetic powder.

The cosmetic products, such as foundations, fards, eye shadows, lipsticks, etc, which are used to make up the skin and the lips are constituted mostly by inorganic powders and by some organic powders.

Applying these products onto the skin surface a thin coloured film is formed which masks the aesthetic defects of the skin and gives it a more uniform appearance.

This film is simply deposited onto the skin surface hence the inorganic/organic cosmetic powders are directly into contact with the superficial layers of the epidermis.

It is well known that the direct contact of inorganic/organic cosmetic powders with the skin can leads to the absorption of the water present on the skin surface, thus altering the natural hydrophilic/lipophilic balance, which could then cause local dehydration effects and consequently create unpleasant feeling by the users

In addition, the dishomogenicity of the used individual powders, having physical characteristics different between them could generate in the final analysis clearly perceptible anaesthetic effects.

It is known the use of silicones to treat and coated cosmetic powders.

For example, the US patent 5,496,544 discloses a cosmetic composition for skin consisting of an anhydrous powder that includes a solid particulate phase mixed with a fatty binder containing a silicone mixture comprising at least one silicone oil, at least one silicone wax, at least one silicone resin, and optionally at least one silicon gum, and optionally at least one phenyldimethicone.

The patent application EP 678015 describes, inter alia, that nonlimiting classes of surface treatment materials useful for treating the pigment particles include silicones. Among the examples of useful silicones as surface treatment materials are included dimethicone, cyclomethicone, dimethiconol, dimethicone copolyol, dimethicone copolyol acetate, dimethicone copolyol butyl ether, dimethicone copolyol methyl ether, and mixtures thereof. Also useful are fluorinated, phenyl-substituted, and amino-substituted derivatives of these silicones.

The US patent 4,578,266 discloses pigmented cosmetic compositions containing an organically substituted polysiloxane by the pigment coated with a hydrophobic silicone.

In view of the state of the art above described, it is an object of the present invention to provide a powder treated in such a way that the contact between the powder and the skin surface is prevented, avoiding dehydration effects and meanwhile generating a good sensory effect.

According to the present invention, such object is achieved by using a cosmetic powder characterised by being constituted by a powder phase coated with silsesquioxane homopolymers and/or copolymers having an average siloxane unit of the general formula R¹ₙSiO_{(4-n)/2}, where each R¹ is independently chosen between a hydrogen atom and a monovalent hydrocarbon group comprising 1 to 10 carbon atoms, wherein more than 80 mole % of R¹ are hydrocarbon groups having carbon atoms included between 3 and 10, n is a value included between 0.8 and 1.2, greater than 50 mole % of the copolymer comprises R¹SiO_{3/2} units, and having a hydroxyl or alkoxy content from 0.2 to 10 by weight.

The object of the invention is also reached with a cosmetic product composition containing one or more coated cosmetic powders according to the present invention.

Thanks to the present invention, it is then possible to provide a coated powder, by means of a particular preparation process described in the following, which allows to the silsesquioxane homopolymers and/or copolymers of networking and hooking onto the powders, by partially or totally modifying the physico-chemical properties, making them highly hydrophobic and highly skin adhesive, hence particularly suitable for the formulation of high quality cosmetic compositions, such as foundations both compacted and liquid, eyeshadows, lipsticks, etc.

Moreover, a cosmetic composition for the face make up such as pressed powder, according to the present invention, is particularly suitable to be applied with a wet applicator, as for example a small sponge.

The features and the advantages of the present invention will be made more evident by the following description of some embodiments, reported as not limitative examples.

The present invention concerns the preparation of cosmetic powders in particular for coloured cosmetic products, coated with silsesquioxane homopolymers and/or copolymers having an average siloxane unit of the general formula R¹ₙSiO_{(4-n)/2}, where each R¹ is independently chosen between a hydrogen atom and a monovalent hydrocarbon group comprising 1 to 10 carbon atoms, wherein more than 80 mole % of R¹ are hydrocarbon groups having carbon atoms included between 3 and 10, n is a value from 1.0 to 1.4, more than 60 mole % of the copolymer comprises R¹SiO_{3/2} units, and having a hydroxyl or alkoxy content from 0.2 to 10% by weight.

Preferably, the silsesquioxane polymer used is one where R is an alkyl chain with length varying from C1 to C10, most preferably C3, wherein the presence of the hydroxyl or alkoxy groups is varying between 0.2 and 10% by weight, for example between 1 and 4% by weight, preferably between 5 and 10% by weight, and more preferably between 6 and 8% by weight. Examples of silsesquioxanes or silicone resins include, but are not limited to, those made from silanes having the following structures: R¹Si(OR⁴)₃, R¹R²Si(OR⁴)₂, and R¹R²R³SiOR⁴ where R¹,R²,R³ are alkyl chains having carbon numbers from C1 to C10, preferably C3-C8; and where R⁴ is an alkyl chain having carbon numbers from C1 to C4. Further, inclusion of silsesquioxanes that also contain Si(OR⁴)₄ are also considered on condition that the use of these tetrafunctional silanes are present from 0 to 20 mole % of the silsesquioxane composition.

The presence of hydroxyl or alkoxy functional groups may allow the networking and the hooking onto the surface of the powders.

According to the present invention, the cosmetic powders coated with silsesquioxane polymers are comprehensive of: powders 0.1-99.9%, preferably 80-99% by weight of the total composition; silsesquioxane polymers 0.05-80%, preferably 1-20% by weight of the total composition.

The powder phase can contain excipients and/or pigments traditionally used in the cosmetic field.

As excipients, as example, the following can be used: talc, mica, kaolin, clays, zinc oxide, calcium carbonate, magnesium carbonate phosphate, calcium bisulphate, starch and its derivatives, nylon, polyethylene, polymers and copolymers of acrylic acid, etc.

As pigments, as example, the following, can be used: iron oxides, chromium oxides and hydroxides, blue and pink ultramarine, manganese violet, titanium dioxide, pearls on mica or bismuth oxychloride substrate, carmine, lacquers and pigments based on organic dyes as per CTFA.

The preparation process for the coating of the powders consistin in using a turbomixer which is fed in a continuous process with the powders to be coated through a loading hopper. Within the mixer, under turbulent stirring the powder is impregnated by means of dosing pumps which spray the coating phase previously prepared mixing it with a solvent.

Solvents are defined as being any common diluent that is capable of providing a fully solvated or dispersed solution of the silsesquioxane resin. Preferred solvents include aliphatic hydrocarbon and low viscosity siloxane fluid polymers. For example, include but are not limited to, isoparaffins with a low molecular weight, decane, dodecane, tridecane, tetradecane, pentadecane, hexadecane, heptadecane and octadecane as well as branched isomers thereof; volatile siloxane fluids such as octamethylcyclotetrasiloxane, decamethylcyclopentasiloxane, etc. Other suitable solvents may include ketones, esters and oils such as paraffinic or naphthenic oils.

The whole preparation process is carried out under nitrogen flux in order to make the atmosphere within the mixer fully inert.

The powders impregnated and collected in special containers, are kept at room temperature for 48 hours and thereafter, they are transferred in a special oven at 80°C where they are left until the complete evaporation of the solvent. In this way, the hooking of the pure resin onto the powders can also be achieved.

Other processing techniques applicable for treating solids are known and are included as part of this invention provided that the basic steps of (1) producing a resin solution, (2) mixing it intimately with the powder and then (3) removing the solvent by evaporating are completed.

A cosmetic composition according to the present invention includes at least a powder coated with silsesquioxane polymers, in combination with other ingredients typical of the cosmetic compositions.

According to the invention, the composition of a cosmetic product for make-up which uses such coated powders, includes: a powder phase comprehensive of coated powders 0.1-99.9%, preferably 40-90% by weight of the total composition; various cosmetically acceptable ingredients 0.1-99.9%, preferably 10-60% by weight of the total composition.

As cosmetically acceptable ingredients, as example are included: oils and fats, silicones, polyurethane derivatives, perfluorinated compounds, other non coated powders, fragrances and aromas, dyes, pigments or other ingredients included in the CTFA dictionary.

The chemical but mostly the physical characteristics of the coating of the present invention allow to coat the powders and the pigments by creating a thin layer (film), with physical properties which mirror the properties of the coating, i.e. flexibility, elasticity, film softness which does not crack. The podwercoating achieved by using other polymers which have different chemical nature, but mostly physical characteristics (e.g. trymethylsiloxysilicate) leads to a layer deposition which is stiff, crystalline and less flexible and give unpleasant sensorial properties to those products which contain it.

In particular, a dry skin feel is perceived, in addition to a sub-optimal colour yield, when the pigments are coated.

The above reported perceived benefits are the results of sensorial tests carried out by a voluntary panel of examiners focussed on the cosmetic properties assessment.

Herebelow some not limitative examples of cosmetic compositions are listed, that comprises powders coated with silsesquioxane homopolymers and/or copolymers having an average siloxane unit of the general formula R¹ₙSiO_{(4-n)/2}, where each R¹ is independently chosen between a hydrogen atom and a monovalent hydrocarbon group comprising 1 to 10 carbon atoms, wherein more than 80 mole % of R¹ are hydrocarbon groups having 3 carbon atoms, n is a value from 0.8 to 1.2, more than 50 mole % of the copolymer comprises R¹SiO_{3/2} units, and having a hydroxyl or alkoxy content from 0:2 to 5% by weight (hereinafter referred to as silsesquioxane polymers).

| **Example n. 1** | |
|---|---|
| *Pressed powder foundation* | |
| *Ingredients* | *%by weight* |
| Talc coated with silsesquioxane polymers | 78.02 |
| Mica coated with silsesquioxane polymers | 5.25 |
| Yellow iron oxide coated with silsesquioxane polymers | 1.50 |
| Red iron oxide coated with silsesquioxane polymers | 0.62 |
| Black iron oxide coated with silsesquioxane polymers | 0.25 |
| Brown iron oxide coated with silsesquioxane polymers | 0.56 |
| Spherical silica coated with dimethicone | 9.00 |
| Dimethicone (DOW CORNING® 1413) | 0.50 |
| Titanum dioxide coated with silsesquioxane polymers | 1.00 |
| Nylon-12/Octlyldodecyl Stearoyl Stearate | 3.00 |
| Preservatives | 0.30 |
| TOTAL | 100.00 |

The formulation provided a high degree of softness, good colour yield, mildness and glide, without any skin dryness.

| **Example n. 2** | |
|---|---|
| *Pressed powder eyeshadow* | |
| *Ingredients* | *% by weight* |
| Talc coated with silsesquioxane polymers | 59.00 |
| Mica coated with silsesquioxane polymers | 4.00 |
| Yellow iron oxide coated with silsesquioxane polymers | 6.50 |
| Red iron oxide coated with silsesquioxane polymers | 3.00 |
| Black iron oxide coated with silsesquioxane polymers | 0.20 |
| Pearls (Titanium-Mica) coated with DMS | 6.80 |
| Pearls (Titanium-Iron oxides) coated with DMS | 12.20 |
| Dimethicone (DOW CORNING® 1413) | 3.00 |
| Cetearyl Octanoate | 3.00 |
| Fluid DOW CORNING® 200/350 | 2.00 |
| Preservatives | 0.30 |
| TOTAL | 100.00 |

The formulation provided a high degree of softness, good colour yield, mildness and glide, and increased wear without any skin dryness.

| **Example n. 3** | |
|---|---|
| *Lipstick* | |
| *Ingredients* | *% by weight* |
| Fat phase for lipsticks | 82.00 |
| Candelilla wax | 1.20 |
| Tridecyl trimellitate | 5.05 |
| Red iron oxide coated with silsesquioxane polymers | 2.70 |
| FD&C Red 7 Al Lake coated with silsesquioxane polymers | 0.55 |
| FD&C Yellow 5 coated with silsesquioxane polymers | 1.30 |
| Pearls (Mica-Titanium dioxide) coated with silsesquioxane polymers | 6.00 |
| Titanium dioxide | 1.20 |
| TOTAL | 100.00 |

The formulation provided a high degree of compatibility with silicone containing bases and better colour yield and glide.

| **Example n. 4** | |
|---|---|
| *Fluid foundation* | |
| *Ingredients* | *% by weight* |
| Microcristalline wax | 1.40 |
| Laureth ― 9 | 0.60 |
| Polygliceryl ― 4- Isostearate | 0.80 |
| Ciclomethicone | 13.90 |
| Silicone derivatives | 20.50 |
| Propylene Glycol | 4.00 |
| Glycerol | 2,00 |
| Sodium Chloride | 1.60 |
| Water | 41.00 |
| Red iron oxide coated with silsesquioxane polymers | 1.40 |
| Black iron oxide coated with silsesquioxane polymers | 0.20 |
| Yellow iron oxide coated with silsesquioxane polymers | 3.20 |
| Titanium dioxide coated with silsesquioxane polymers | 8.20 |
| Talc coated with silsesquioxane polymers | 1.20 |
| TOTAL | 100.00 |

The formulation provided improved homogeneity in the coated pigments dispersion in Water/Silicone emulsion type, and further ease of application and softness.

## Claims

1. A cosmetic powder **characterised by** being constituted by a powder phase coated with silsesquioxane homopolymers and/or copolymers having an average siloxane unit of the general formula R¹ₙSiO_{(4-n)/2}, where each R¹ is independently chosen between a hydrogen atom and a monovalent hydrocarbon group comprising 1 to 10 carbon atoms, wherein more than 80 mole % of R¹ are hydrocarbon groups having from 3 to 10 carbon atoms, n is a value comprised between 1.0 and 1.4, more than 60 mole % of the copolymer comprises R¹SiO_{3/2} units, and having a hydroxyl or alkoxy content from 0.2 to 10% by weight.

2. A cosmetic powder according to claim 1 **characterised by** including the following ingredients:
0.1-99.9% by weight of said cosmetic powder phase;
0.05-80% by weight of said silsesquioxane homopolymers and/or copolymers.

3. A cosmetic powder according to claim 1 or 2 **characterised by** including the following ingredients:
80-99% by weight of said cosmetic powder phase;
1-20% by weight of said silsesquioxane homopolymers and/or copolymers.

4. A cosmetic powder according to any of the claims 1-3 wherein such silsesquioxane homopolymers and/or copolymers are those in which R¹ are propyl groups.

5. A cosmetic powder according to any of the preceding claims, in which said cosmetic powder phase comprises at least a pigment.

6. A cosmetic powder according to any of the preceding claims, in which said cosmetic powder phase comprises at least an excipient.

7. A composition of a cosmetic product containing one or more cosmetic powders coated according to any of the claims 1-6, **characterised in that** it contains other cosmetically acceptable ingredients.
